# EUROPEAN PATENT APPLICATION

(11) **EP 4 249 958 A1**
(43) Date of publication of application: **27.09.2023**
(21) Application number: 21894727.3
(22) Date of filing: 18.11.2021
(51) Int. Cl.: G01T 1/105, G01T 1/20, G03B 42/02, A61B 6/00

(54) **IMAGE PROCESSING SYSTEM AND IMAGE PROCESSING DEVICE**

(30) Priority: 20.11.2020 JP 2020193778; 20.11.2020 JP 2020193783
(71) Applicant: J. MORITA MANUFACTURING CORPORATION, Fushimi-ku, Kyoto-shi, Kyoto 6128533 (JP)
(72) Inventor: YOSHIOKA, Tadashi, Kyoto-shi, Kyoto 612-8533 (JP); SUGIHARA, Yoshito, Kyoto-shi, Kyoto 612-8533 (JP)
(74) Representative: Müller Hoffmann & Partner
(86) International application number: PCT/JP2021/042467
(87) International publication number: WO 2022/107855

(57) **Abstract**

imaging plate on which a radiographic image is recorded to generate an X-ray image. The image processing system performs signal processing on the total output area included in the read signal such that the degree of change in density regarding a partial attention region according to the purpose of observation is larger than the degree of change in density regarding the non-attention region other than the attention region.

## Description

### Technical Field

The present disclosure specifically relates to image processing and an image processing apparatus that processes a read signal read from an imaging plate on which a radiographic image is recorded to generate an X-ray image.

### Background Art

Conventionally, there is known a technique of reading an imaging plate (abbreviated as IP) on which latent images are recorded by exposure to X-rays and performing image processing of X-ray images (see, for example, Patent Document 1). The reading device irradiates the imaging plate with laser light, detects stimulated emission excited by the irradiation of the laser light, performs processing such as logarithmic conversion amplification and A/D conversion, and outputs a digital signal.

### Citation List

### Patent Literature

[Patent Document 1] Japanese Unexamined Patent Publication No. 2010-172362

### Summary of Invention

### Technical Problem

In the above-described conventional technique, a read signal obtained by detection of stimulated emission is subjected to processing such as logarithmic conversion amplification and A/D conversion. However, in the conventional technique, a blurred image is generated depending on the purpose of observation. The blurred image means an image in which the contrast of a portion to be observed is not clear and is fuzzy.

The present disclosure describes the image processing system that can improve the contrast of a region to be observed in an obtained X-ray image.

### Solution to Problem

One aspect of the present disclosure is an image processing system that generates an X-ray image by processing a read signal read from the imaging plate on which a radiographic image is recorded, wherein signal processing is performed on a total output area included in the read signal such that a degree of change in density regarding a partial attention region according to an observation purpose is larger than a degree of change in density regarding a non-attention region other than the attention region.

According to this the image processing system, in the total output area included in the read signal, the degree of change in density becomes large only for the partial attention region corresponding to the purpose of observation. Therefore, in the obtained X-ray image, the contrast of the portion to be observed is improved.

The image processing system may perform the signal processing by logarithmic conversion. Logarithmic conversion makes it easier to understand the change in density even when the intensity of the dose is low, for example. This provides a clinically manageable X-ray image.

The image processing system may set the attention region and the non-attention region such that a dose intensity range corresponding to the attention region is existing on a lower dose side than a dose intensity range corresponding to the non-attention region. In this case, the contrast of the low dose side (attention region) is emphasized at the expense of the contrast of the high dose side (non-attention region). Thus, a high contrast can be achieved in a region where the intensity of the dose is low.

The image processing system may set the attention region and the non-attention region such that a dose intensity range corresponding to the attention region is existing on a higher dose side than a dose intensity range corresponding to the non-attention region. In this case, the contrast of the high dose side (attention region) is emphasized at the expense of the contrast of the low dose side (non-attention region). Thus, a high contrast can be achieved in a region where the intensity of the dose is high.

The image processing system may set the attention region and the non-attention region such that the non-attention region includes a first non-attention region having a dose intensity range existing on a lower dose side than a dose intensity range corresponding to the attention region and a second non-attention region having a dose intensity range existing on a higher dose side than the dose intensity range corresponding to the attention region. In this case, the attention region corresponding to a desired dose intensity range is set, and the contrast in the attention region is emphasized.

The image processing system may perform the signal processing such that the non-attention region is represented with a constant density by eliminating a degree of change in density of the non-attention region. In this case, the attention region and the non-attention region can be further modulated. The image portion corresponding to the non-attention the attention region becomes an image having uniform density and no contrast, and the image portion corresponding to the region becomes an image having a large concentration change and clearer contrast.

The image processing system may perform the signal processing on an analog signal before an analog-digital conversion. When the signal processing is performed on a digital signal, fine differences in output values may disappear. By performing the signal processing on the attention region before (prior to) the analog-digital conversion, the difference of the analog signal can be reflected in density in the X-ray image.

Another aspect of the present disclosure is an image processing system that generates an X-ray image by processing a detection signal obtained by radiography, wherein signal processing is performed on a total output area included in the detection signal such that a degree of change in density regarding a partial attention region according to an observation purpose is larger than a degree of change in density regarding a non-attention region other than the attention region.

According to this the image processing system, in the total output area included in the detection signal, the degree of change in density becomes large only for the partial attention region corresponding to the purpose of observation. Therefore, in the obtained X-ray image, the contrast of the portion to be observed is improved.

### Effects of Invention

According to some aspects of the present disclosure, it is possible to improve contrast in a region desired to be observed in an obtained X-ray image.

### Brief Description of Drawings

FIG. 1 is a diagram illustrating a schematic configuration of the image processing system according to an embodiment of the present disclosure.
FIG. 2 is a diagram showing a more specific configuration of the image processing system shown in FIG. 1.
FIG. 3 is a block diagram showing a configuration example of an image processing unit (image processing apparatus).
FIG. 4 is a flow diagram of the basic processing performed in the image processing system.
FIG. 5 is a flow diagram illustrating one example of processing performed in the image processing system.
FIG. 6(a) is a diagram showing characteristics of the analog signal, and FIG. 6(b) is a diagram showing a degree of change of an output after signal processing.
FIG. 7(a) is a diagram showing characteristics of the analog signal, and FIG. 7(b) is a diagram showing a degree of change of an output after the signal processing.
FIG. 8 is a view showing an aluminum stairsteps phantom having a plurality of steps of thickness.
FIGS. 9(a) and 9(b) are diagrams illustrating the degree of change in density when the signal processing according to the present disclosure is not performed (no revision).
FIGS. 10(a) and 10(b) are diagrams showing the degree of change in density when the attention region is set in a low dose side (X-ray high absorption region), and FIG. 10(c) is a diagram showing an example of an X-ray image of a dental practice in that case.
FIGS. 11(a) and 11(b) are diagrams showing the degree of change in density when the attention region is set in a high dose side (low X-ray absorption region), and FIG. 11(c) is a diagram showing an example of an X-ray image of the dental practice in that case.
FIG. 12(a) is a diagram showing a change in each step in the case of no revision, and FIG. 12(b) is a diagram showing a change in each step in the case of revision.
FIG. 13 is a block diagram showing another configuration example of the image processing unit (image processing apparatus).
FIG. 14(a) is a diagram showing characteristics of the analog signal, and FIG. 14(b) is a diagram showing a degree of change of an output subjected to the signal processing according to the configuration example of FIG. 13.
FIG. 15 is a flow diagram illustrating another example of processing performed in the image processing system.
FIG. 16(a) is a diagram showing an example of an object, and FIGS. 16(b) and 16(c) are diagrams showing examples of intensity detection.
FIG. 17(a) is a diagram showing characteristics of the analog signal, and FIG. 17(b) is a diagram showing a degree of change of an output after the signal processing according to the configuration example of FIG. 13.
FIG. 18(a) is a diagram showing characteristics of the analog signal, and FIG. 18(b) is a diagram showing a degree of change of an output after the signal processing according to the configuration example of FIG. 13.

### Description of Embodiments

Hereinafter, embodiments of the present disclosure will be described with reference to the drawings. In the description of the drawings, the same elements are denoted by the same reference numerals, and redundant description is omitted.

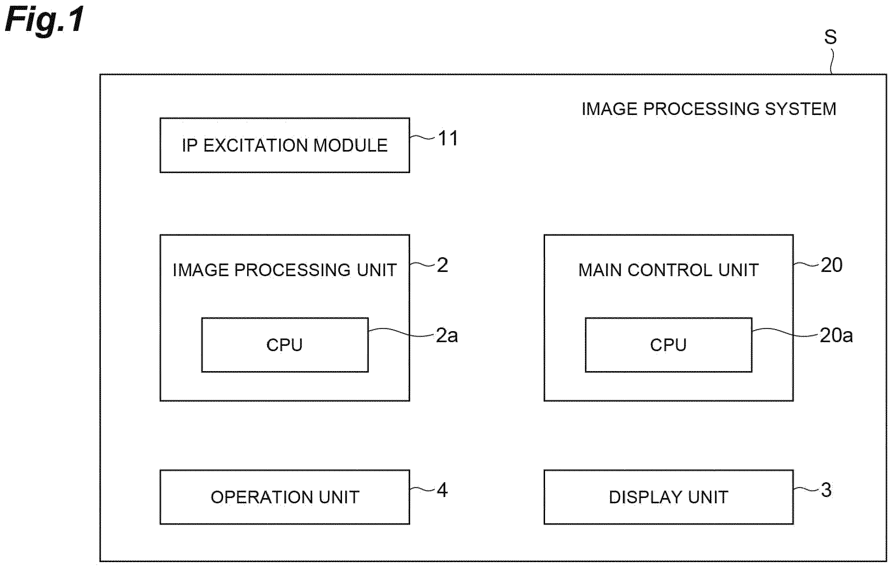

With reference to FIG. 1, a basic configuration of an image processing system S of the present embodiment will be described. The image processing system S is used, by way of example, for radiography in a dental practice. In the dental practice, an imaging plate 100 is placed on an oral cavity of the patient and X-rays in the oral cavity are performed. Radiographic images are recorded on the imaging plate 100. An imaging plate may also be referred to as an imaging plate, and thus may be referred to as the imaging plate.

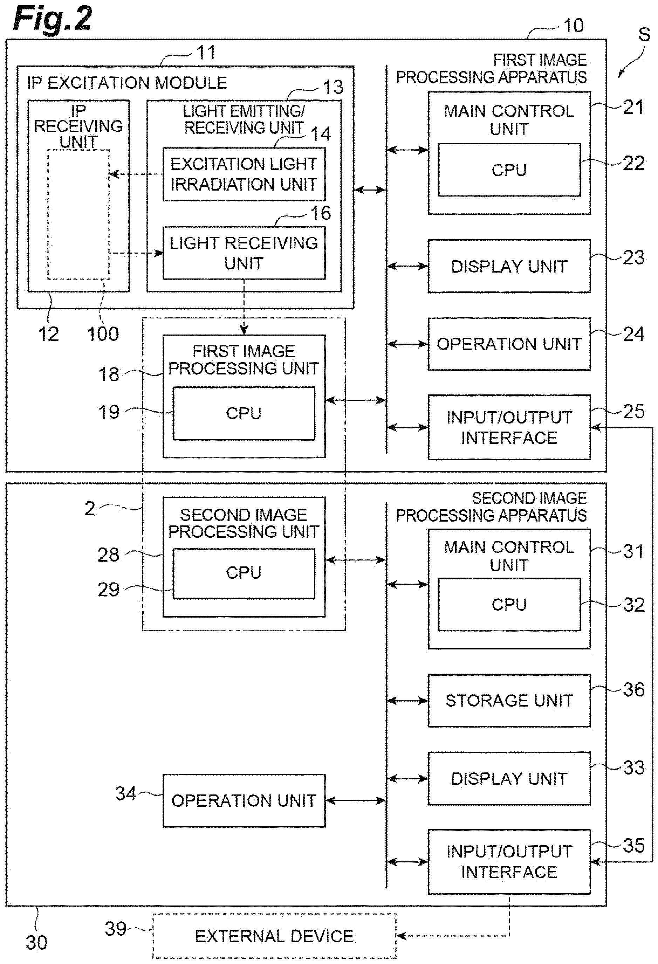

As shown in FIG. 1, the image processing system S is a system that generates an X-ray image by processing a read signal read from the imaging plate 100 (see FIG. 2) on which a radiographic image is recorded. The image processing system S comprises an IP excitation module 11, an image processing unit 2, a main control unit 20, an operation unit (physical interface) 4 and a display unit (display) 3. The IP excitation module 11 illuminates the imaging plate 100 's an excitation light, scans the imaging plate 100, and detects stimulated emissions occurring in the imaging plate 100. The IP excitation module 11 includes, for example, a photodiode array and an amplifier, and outputs a read signal of the radiographic image based on the obtained luminance information. As the IP excitation module 11, a known configuration can be adopted. The IP excitation module 11 may be configured as a set of the excitation light source and a stimulated emission scanner, and may be expressed as a light source a scanner 11. The main control unit 20 has a CPU 20a and controls each part of the image processing system S. The operation unit 4 includes an operation button, a touch panel display, or the like, and accepts an operation by the user. The operation unit 4 outputs a signal according to the input operation content to the main control unit 20. The image processing unit 2 has a CPU 2a and is controlled by the main control unit 20 to perform various operations related to image processing. The image processing unit 2 performs an analog-digital conversion, synthesis processing, and the like on the read signal to generate an X-ray image. The main control unit 20 causes the display unit 3 to display an X-ray image. The image processing unit 2 may also serve as the main control unit 20. The main control unit 20 may be composed of circuitry and may be described as a main control circuitry 20. The image processing unit 2 may be composed of circuitry and may be described as a sub-image processing circuitry 2.

The image processing system S may integrally comprise the arrangement described above. For example, the IP excitation module 11 and a part of the image processing unit 2 may be integrated, and the other part of the image processing unit 2 and the display unit 3 may be integrated, and these may be communicably connected by wiring, a network, or the like (see FIG. 2). In this case, the operation unit 4 may be provided on both sides. The image processing system S may comprise a circuitry. The image processing system S may be referred to as an image processing circuitry S. The components of the image processing system S may be integrated into, for example, a case or a housing. In that case, the image processing system S is configured as an "image processing apparatus". That is, the image processing system S may be expressed as an image processing apparatus S. Next, a specific configuration example of the image processing system S will be described with reference to FIG. 2. In the following description, the imaging plate 100 is abbreviated as IP 100.

The image processing system S shown in FIG. 2 comprises a first image processing apparatus 10 and a second image processing apparatus 30. The first image processing apparatus 10 and the second image processing apparatus 30 are connected by wiring, a network, or the like, and can communicate information with each other via input/output interfaces 25, 35. The first image processing apparatus 10 comprises an IP excitation module 11, a first an image processing unit 18 comprising CPU 19, a main control unit 21 comprising CPU 22, a display unit 23, an operation unit 24, and the input/output interface 25. The second image processing apparatus 30 includes a second image processing unit 28 including a CPU 29, a main control unit 31 including a CPU 32, a storage unit (memory) 36, a display unit (display) 33, an operation unit (physical interface) 34, and an input/output interface 35. The display unit 23 and the display unit 33 correspond to the display unit 3 in FIG. 1. The operation unit 24 and the operation unit 34 correspond to the operation unit 4 in FIG. 1.

The first image processing apparatus 10 may be configured as a circuitry and may be referred to as a first image processing circuitry 10. The second image processing apparatus 30 may be configured as a circuitry and may be referred to as a second image processing circuitry 30. The first image processing unit 18 may be configured as a circuitry and may be referred to as a first sub-image processing circuitry 18. The second image processing unit 28 may be constituted by a circuitry and may be expressed as a second sub-image processing circuitry 28. The main control unit 21 may be constituted by a circuitry and may be expressed as the first main control circuitry 21. The main control unit 31 may be constituted by a circuitry and may be expressed as the second main control circuit 31. The CPU 19 may be referred to as a first image processing processor 19. The CPU 22 may be referred to as the first main control processor 22. The CPU 29 may be referred to as a second image processing processor 29. The CPU 32 may be referred to as the second main control processor 32.

The IP excitation module 11 includes an IP receiving unit 12 that receives IP 100, an excitation light irradiation unit (the excitation light source) 14 that irradiates the excitation light to IP 100 in the IP receiving unit 12, and a light receiving unit (optical receiver) 16 that receives light generated by the excitation light irradiation unit 14. The light receiving unit 16 outputs a read signal of the radiographic image to the first image processing unit 18. The first image processing unit 18 and the second image processing unit 28 correspond to the image processing unit 2 in FIG. 1. The first image processing unit 18 and the second image processing unit 28 perform a predetermined image processing step. The processing performed by the first image processing unit 18 and the second image processing unit 2 8 will be described later. The second image processing apparatus 30 displays the X-ray image output from the second image processing unit 28 on the display unit 33 and outputs the X-ray image to an external device 39.

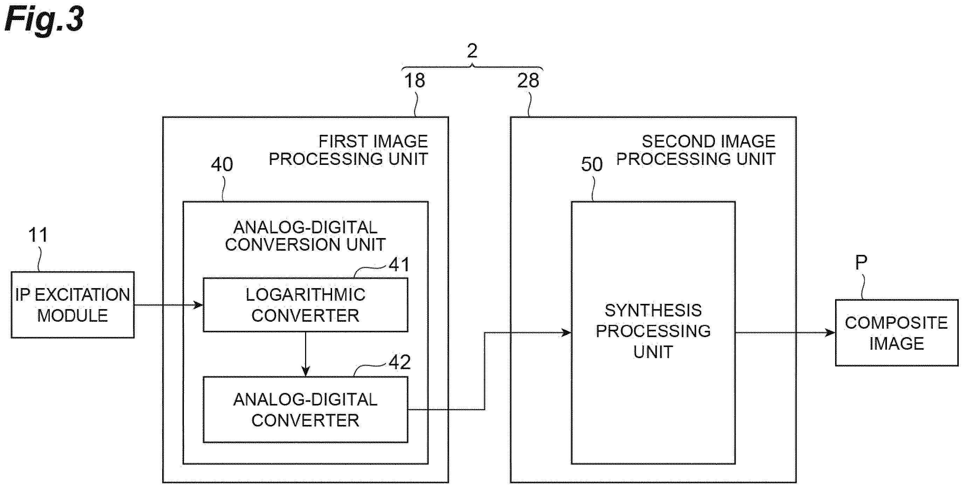

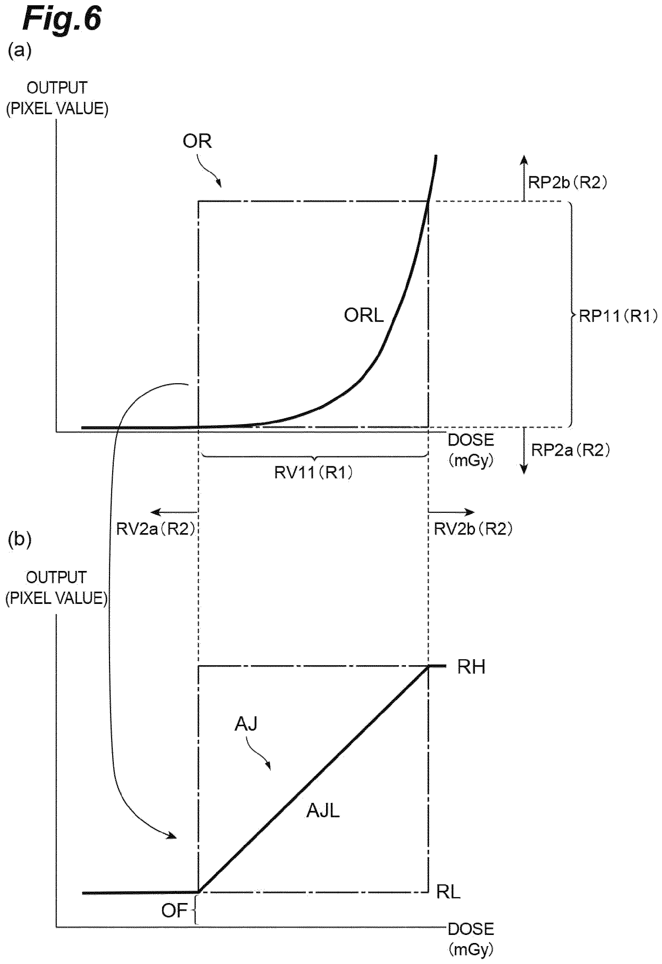

A configuration example in the image processing unit 2 will be described with reference to FIG. 3. As shown in FIG. 3, the first image processing unit 18 of the present embodiment includes an analog-digital conversion unit 40 that performs the analog-digital conversion on the read signal output from the IP excitation module 11. The first image processing unit 18 has the analog-digital conversion unit 40 and thus performs signal processing on an analog signal before the analog-digital conversion. Particularly, in the present embodiment, the analog-digital conversion unit 40 sets partial attention regions RV11 and RP11 (R1) according to the purpose of observation and a non-attention region RV2a, RP2a, RV2b, and RP2b (R2) other than an attention region R1 in the total output area included in the read signal (see FIG. 6(a)). The non-attention region RV2a and RP2a are dose intensity ranges existing on the lower dose side than a dose intensity range corresponding to the attention region R1, and a non-attention region RV2b and RP2b are dose intensity ranges existing on the higher dose side than the dose intensity range corresponding to the attention region R1. For example, in the non-attention region, the first non-attention region RV2a and RP2a having lower output values may be determined to be represented by a uniform adjusted output value (for example, white), and the second non-attention region RV2b and RP2b having higher output values may be determined to be represented by a uniform adjusted output value (for example, black). For the attention region R1, a processing of optimizing the contrast is performed (see FIG. 6(b)). Note that the first non-attention region RP2a may be an offset value OF. According to such signal processing (image processing), it is possible to avoid a burden of unnecessary calculation and to effectively use a limited number of bits in calculation of the processor. In the graph, an element on the vertical axis is expressed by insertion of a character P, and an element on the horizontal axis is expressed by insertion of a character V. The analog-digital conversion unit 40 may be composed of circuitry and may be described as an analog-digital conversion circuitry 40.

The pattern of the original output with respect to the dose, which is not subjected to the output adjustment processing according to the present configuration, is referred to as an original output pattern OR, and this the original output pattern OR is indicated by an original output pattern line ORL on the graph as illustrated in FIG. 6(a). The image processing unit 2 performs the signal processing on the analog signal. A pattern of an output with respect to a dose, which is adjusted after the signal processing, is referred to as an adjustment output pattern AJ. This the adjustment output pattern AJ is indicated by an adjusted output pattern line AJL on the graph as shown in FIG. 6(b). In the original output pattern OR, a low dose region includes many regions in which a change in the output value with respect to a change in the dose value is small, and a high dose region includes many regions in which a change in the output value with respect to a change in the dose value is steep.

Here, it is conceivable to perform the signal processing so that the degree of change becomes large (steep) in the adjustment output pattern AJ with respect to a region in which the change of the output value with respect to the change of the dose value in the original output pattern OR is poor, that is, a region in which the degree of change in density is poor.

More preferably, it is conceivable to perform the signal processing so that the degree of change becomes small (gentle) in the adjustment output pattern AJ with respect to a region where the change of the output value with respect to the change of the dose value in the original output pattern OR is steep, that is, a region where the degree of change in density is steep. FIG. 6(b) shows an example of a linear adjustment pattern to which these processes are applied. Although the adjustment output pattern AJ 's an upper limit value RH and a lower limit value RL may be determined as appropriate, the upper limit value RH and the lower limit value RL may be matched to the minimum and maximum values of the attention region R1 in the original output pattern OR as shown.

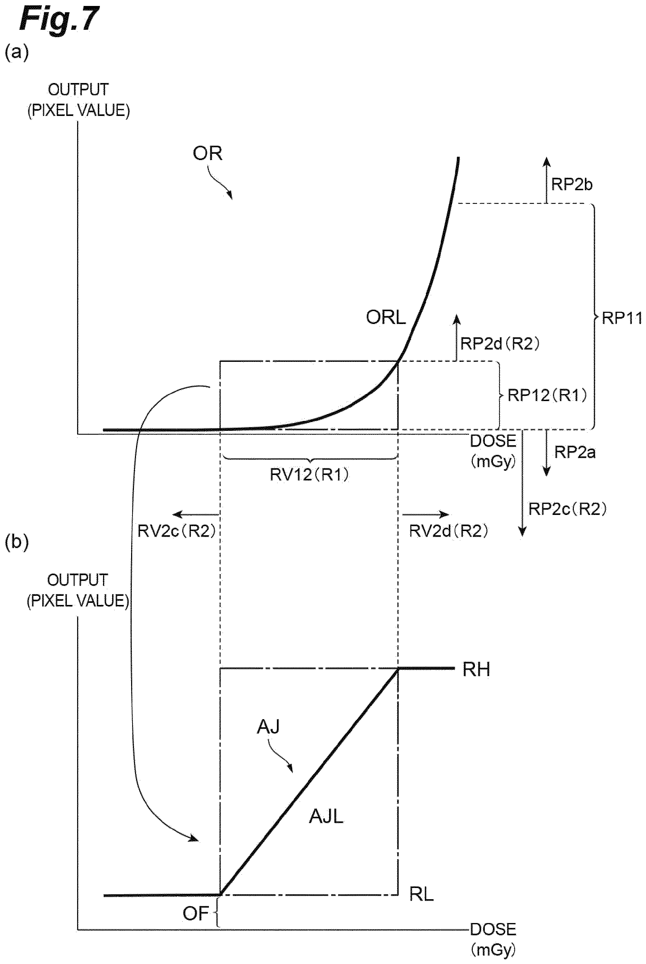

In contrast to the adjustment pattern that is linear over a wide range of regions as shown in FIG. 6, it is conceivable to limit the attention region R1 and perform the signal processing such that the degree of change in density for the non-attention the attention region R1 is greater (steeper) than the degree of change in density for the non-attention region. FIG. 7(b) shows an example in which such adjustment processing is performed. In FIG. 7, the range of an attention region RV12 and RP12 (R1) is more limited to the low-dose region than the example of R1 shown in FIG. 6. The analog-digital conversion unit 40 performs the signal processing such that the degree of change in density for an attention region is greater (steeper) than the degree of change in density for a non-attention region R2 (RV2c, RP2c, RV2d, RP2d). The degree of change in density for the non-attention the attention region is less severe than the degree of change in density for the region. The upper limit value RH and the lower limit value RL of the adjustment output pattern AJ may be determined as appropriate. The upper limit value RH and the lower limit value RL may be matched to the upper limit value and lower limit value widths of the adjustment output pattern AJ of FIG. 6 as shown. The adjustment processing will be described in detail later.

The analog-digital conversion unit 40 includes a logarithmic converter 41 and a first an analog-digital converter 42. The logarithmic converter 41 performs logarithmic conversion on the read signal. The signal processing for making the degree of change in density steep or gentle with respect to the attention region and the non-attention region described above is performed before the analog-digital conversion by the analog-digital converter 42. The signal processing may be performed before the logarithmic conversion by the logarithmic converter 41 or after the logarithmic conversion.

The second image processing unit 2 8 contains a synthesis processing unit 50. The synthesis processing unit 50 receives the digital signal output from the first analog-digital converter 42 and outputs an X-ray image data (data of a composite image P). The synthesis processing unit 50 may be composed of circuitry and may be described as a synthesis processing circuitry 50.

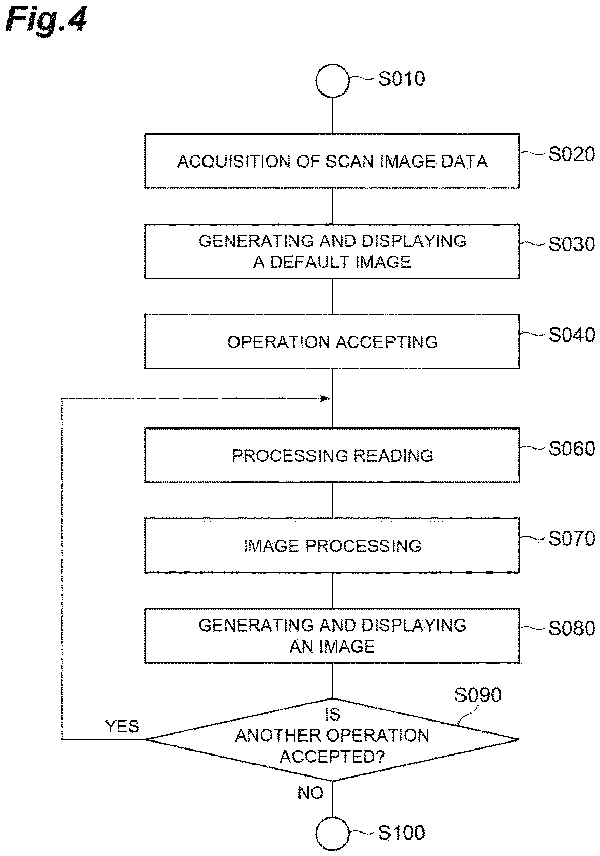

Next, with reference to FIG. 4, a basic processing executed in the image processing system S will be described. As shown in FIG. 4, the image processing unit 2 (see FIG. 1) acquires a scan image data (read signal) from the IP excitation module 11 (step S020). The image processing unit 2 generates and displays a default image (step S030). Subsequently, when the operation unit 4 is operated, the main control unit 20 accepts the operation (step S040). The main control unit 20 reads the processing (step S060). The image processing unit 2 executes image processing based on the acquired scan image data (step S070). The image processing unit 2 generates an X-ray image, and the main control unit 20 displays the X-ray image in the display unit 3 (step S080). The main control unit 20 accepts another operation (step S090). If another operation is accepted, the main control unit 20 returns to the processing in step S060. The scanned image data may be stored in the storage unit 36, and the scanned image data may be acquired from the storage unit 36 when the processing returns to step 060. If no other operation is accepted, the image processing unit 2 ends the series of processes (step S100). The treatment carried out by the image processing unit 2 is carried out by the first image processing unit 18 and / or performed by the first 18 and / or the second image processing unit 2 8. the image processing system S The treatment performed by the main control unit 20 is performed by the main control unit 21 and / or the main control unit 31 in the image processing system S shown in FIG. 2.

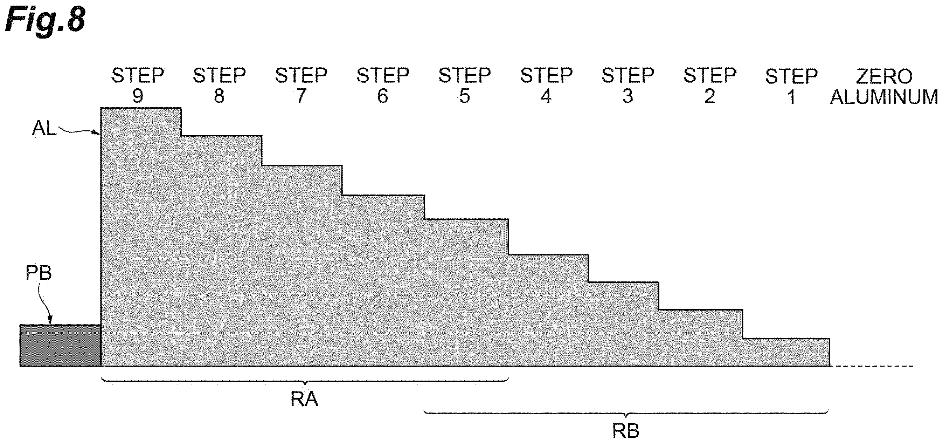

Here, a concept important in the image processing system S according to the present disclosure, that is, a difference in transmission dose (dose intensity) of X-rays according to an observation area of an object will be described with reference to FIG. 8. As shown in FIG. 8, an aluminum stairsteps phantom AL may be provided that simulates a radiographic object (e.g., patient teeth or oral portion). In the aluminum stairsteps phantom AL, for example, aluminum plates are stacked to form stairsteps such that the thicknesses of aluminum vary over 10 steps including zero aluminum. In the portion where the aluminum is thick, more X-rays are absorbed and a smaller X-ray transmission dose is obtained. In the portion where the aluminum is thin, less X-rays are absorbed and a larger X-ray transmission dose is obtained. In the aluminum stairsteps phantom AL, for example, a low dose region RA (high X-ray absorption region) and a high dose region RB (low X-ray absorption region) are assumed.

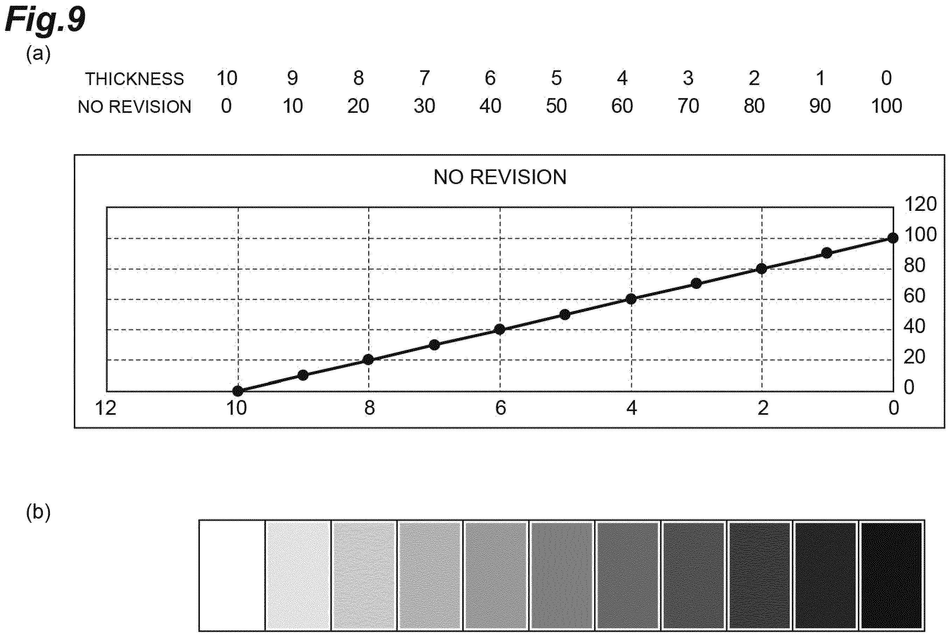

When the aluminum stairsteps phantom AL is X-rayed and the processing including logarithmic conversion as described with reference to FIG. 6 is performed, as shown in FIG. 9(a), for example, the dose intensity tends to increase stepwise as the depth decreases. If the thickness changes continuously rather than stepwise, the dose intensity may change linearly with the thickness. As described above, in normal image processing (stepwise processing), a difference in brightness and darkness occurs evenly over the total output area. In the present embodiment, differences in brightness and darkness are applied only to the attention region of interest.

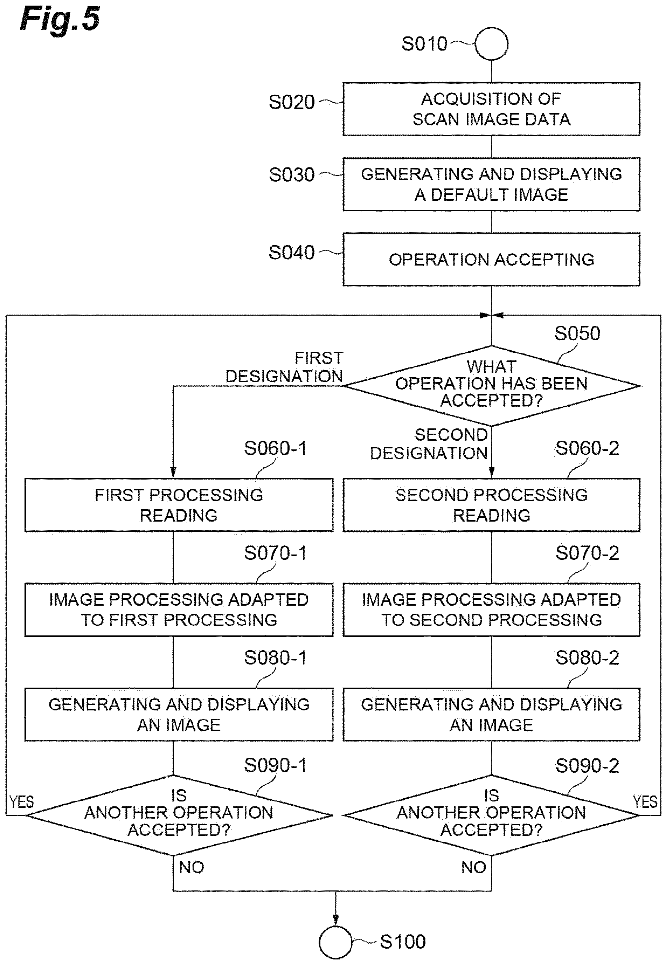

Characteristic and specific processing in the image processing system S of the present embodiment will be described with reference to FIG. 5 and subsequent figures. Description of the same processing as that in FIG. 4 will be omitted. As shown in FIG. 5, the image processing unit 2 executes different processes according to the content of the operation input in step S040. The main control unit 20 determines what operation has been accepted in step S040 (step S050). The types of operations that may be accepted may include a first designation or a second designation. The first designation is an operation in which, for example, periodontal region, end, caries, or the like is designated as a partial attention region according to the purpose of observation. The second designation is an operation in which, for example, front teeth or molar is designated as a partial attention region according to the purpose of observation.

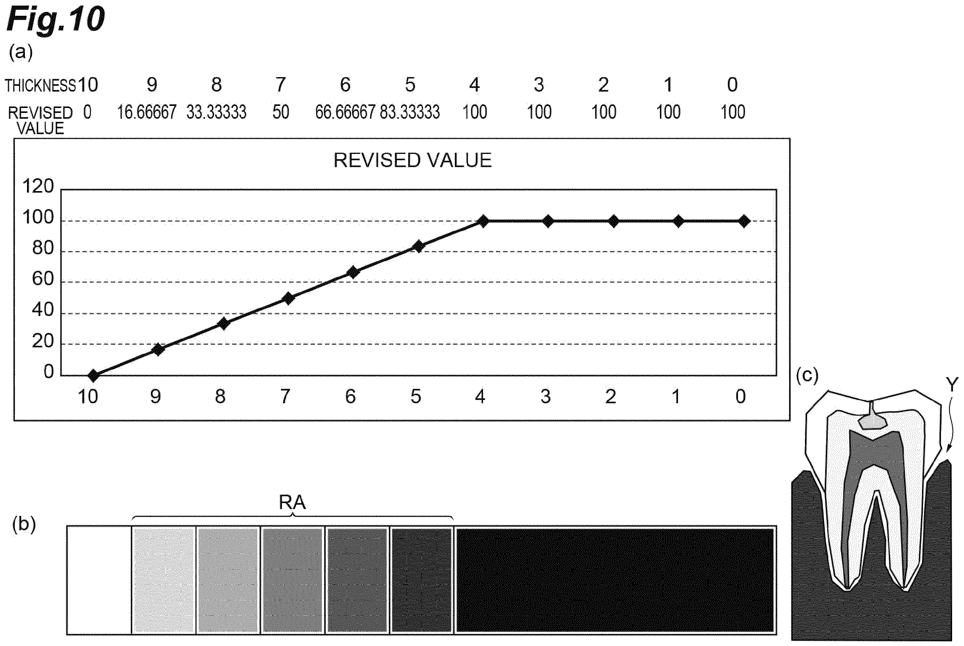

When the first designation is accepted, the main control unit 20 reads out the first processing (step S060-1). The image processing unit 2 executes image processing adapted to the first processing (step S070-1). In step S070-1, the image processing unit 2 sets the attention region and the non-attention region so that the dose intensity range corresponding to the attention region is existing on the lower dose side than the dose intensity range corresponding to the non-attention region. For example, the image processing unit 2 sets a region of less than 60 in the output shown in FIG. 9, that is, the low dose region RA (X-ray high absorption region) shown in FIGS. 8 and 10 to the attention region. The image processing unit 2 sets a region of 60 or more in the output shown in FIG. 9, that is, a region other than the low dose region RA shown in FIGS. 8 and 10 as the non-attention region. The image processing unit 2 may perform the signal processing so that the non-attention region is represented with a constant density by eliminating a degree of change in density regarding the non-attention region. That is, the image processing unit 2 may perform logarithmic conversion and the analog-digital conversion on only the analog signal in the attention region. For example, the analog-digital conversion unit 40 of the first image processing unit 18 performs the image processing described above. The image processing unit 2 generates an X-ray image adapted to the first processing, and the main control unit 20 displays the X-ray image in the display unit 3 (step S080-1). The main control unit 20 accepts another operation (step S090-1). If another operation has been accepted, the main control unit 20 returns to the determination processing of step S050.

When the first designation is accepted, as illustrated in FIG. 10(a), the first image processing unit 18 performs the signal processing such that the degree of change in density regarding the low dose region RA is greater than the degree of change in density regarding regions other than the low dose region RA (see FIG. 10(b)). In this signal processing, when the horizontal axis represents the dose intensity of the detected X-rays and the vertical axis represents the output (pixel value), the degree of change in density regarding the low dose region RA is steeper than the degree of change in density regarding a region other than the low dose region RA. In a high dose side region which is a non-attention region, an output (pixel value) is represented with a constant density. As shown in FIG. 10(b), the region of the high dose side which is the non-attention region is represented by uniform black in the X-ray image. In this way, a dose intensity (value described by mGy) obtained as the analog signal is not evenly divided by a limited number of bits (65,535 in the case of 16 bits), but the dose intensity is evenly divided in the low dose region RA, which is the attention region. Therefore, the contrast resolution of the region to be viewed is optimized according to the purpose of observation. For example, as shown in FIG. 10(c), such image processing is suitable for observation of a tooth neck Y. For example, while the details of a high dose region, such as a periodontal region, may be obscured, caries and the like in the tooth neck Y may be easily discovered.

When the second designation is accepted, the main control unit 20 reads out the second processing (step S060-2). The image processing unit 2 executes image processing adapted to the second processing (step S070-2). In step S070-2, the image processing unit 2 sets the attention region and the non-attention region so that the dose intensity range corresponding to the attention region is existing on the higher dose side than the dose intensity range corresponding to the non-attention region. For example, the image processing unit 2 sets the region equal to or higher than an output 40 shown in FIG. 9, that is, the high dose region RB (low X-ray absorption region) shown in FIGS. 8 and 10 to the attention region. The image processing unit 2 sets a region less than the output 40 shown in FIG. 9, that is, a region other than the high dose region RB shown in FIGS. 8 and 10, as the non-attention region. The image processing unit 2 may perform the signal processing so that the non-attention region is represented with a constant density by eliminating a degree of change in density regarding the non-attention region. That is, the image processing unit 2 may perform logarithmic conversion and the analog-digital conversion on only the analog signal in the attention region. The above-described image processing is executed by the analog-digital conversion unit 40 of the first image processing unit 18, for example. The image processing unit 2 generates an X-ray image suitable for the second processing, and the main control unit 20 displays the X-ray image in the display unit 3 (step S080-2). The main control unit 20 accepts another operation (step S090-2). If another operation has been accepted, the main control unit 20 returns to the determination processing of step S050.

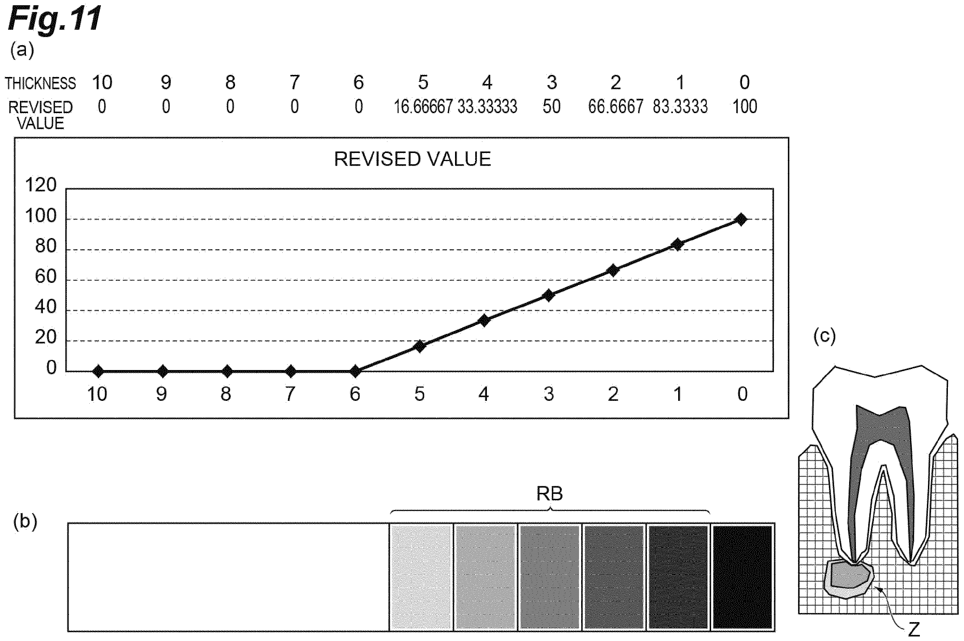

When the second designation is accepted, as illustrated in FIG. 11(a), the first image processing unit 18 performs the signal processing such that the degree of change in density regarding the high dose region RB is greater than the degree of change in density regarding regions other than the high dose region RB (see FIG. 11(b)). In this signal processing, when the horizontal axis represents the dose intensity of the detected X-rays and the vertical axis represents the output (pixel value), the degree of change in density regarding the high dose region RB is steeper than the degree of change in density regarding the region other than the high dose region RB. In a low dose side region which is a non-attention region, an output (pixel value) is represented with a constant density. As shown in FIG. 11(b), the region of the low dose side which is the non-attention region is represented by uniform white in the X-ray image. In this way, the dose intensity (value described by mGy) obtained as the analog signal is not evenly divided by a limited number of bits (65,535 in the case of 16 bits), but the dose intensity is evenly divided in the high dose region RB, which is the attention region. Therefore, the contrast resolution of the region to be viewed is optimized according to the purpose of observation. For example, as shown in FIG. 11(c), such image processing is suitable for observation around an apical portion Z, and details of a low-dose region such as a cervical region become unclear, but an apical lesion or the like around the apical portion Z can be easily found.

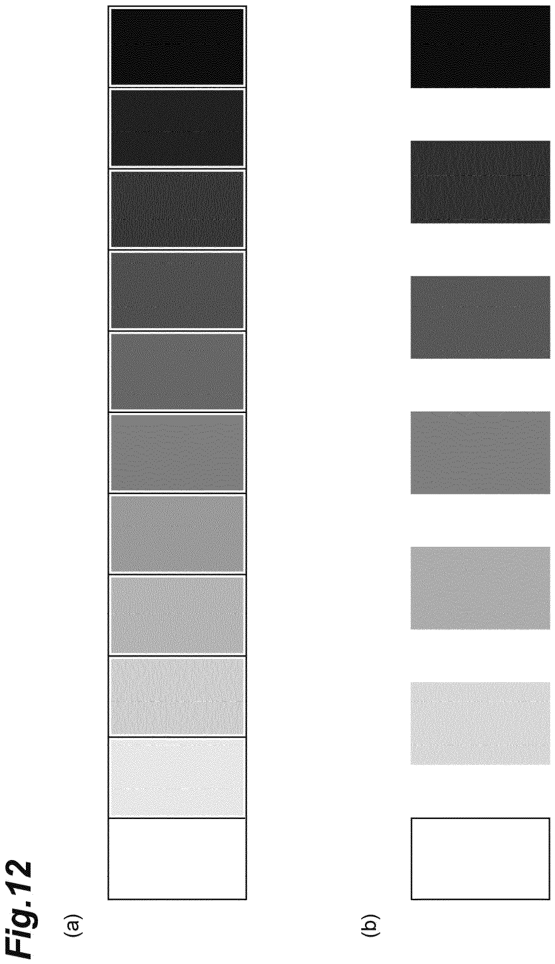

As described above, due to the signal processing by the first image processing unit 18, in the X-ray image, the degree of change in density in the attention region becomes steep. This can be easily understood from the steep degree of change in density as shown in FIG. 12(b) with respect to the uniform degree of change in density as shown in FIG. 12(a) in which the aluminum stairsteps phantom AL is shown as an example.

According to the image processing system S of the present embodiment, the degree of change in density is large only for a partial attention region corresponding to the purpose of observation in the total output area included in the read signal. Therefore, in the obtained X-ray image, the contrast of the portion to be observed is improved.

The image processing system S may perform the signal processing by logarithmic conversion. Logarithmic conversion makes it easy to understand a change in density even when the intensity of the dose is low as in the low dose region RA, for example. This provides a clinically manageable X-ray image.

As shown in FIG. 10(a), the attention region and the non-attention region may be set such that the dose intensity range corresponding to the attention region is existing on a lower dose side than the dose intensity range corresponding to the non-attention region. As shown in FIG. 10(b), the contrast of the low dose side (attention region) is emphasized at the expense of the contrast of the high dose side (non-attention region). Thus, a high contrast can be achieved in a region where the intensity of the dose is low. When the image processing system S is applied to the dental practice, as shown in FIG. 10(c), it is effective for finding caries in the observation of the tooth neck Y.

As illustrated in FIG. 11(a), the attention region and the non-attention region may be set such that the dose intensity range corresponding to the attention region is existing on a higher dose side than the dose intensity range corresponding to the non-attention region. As shown in FIG. 11(b), the contrast of the high dose side (attention region) is emphasized at the expense of the contrast of the low dose side (non-attention region). Thus, a high contrast can be achieved in a region where the intensity of the dose is high. When the image processing system S is applied to the dental practice, as shown in FIG. 11(c), it is effective for finding an apical lesion in the observation of the apical portion Z.

The image processing system S performs the signal processing so that the non-attention region is represented with a constant density by eliminating a degree of change in density of the non-attention region. As a result, modulation in the attention region and the non-attention region can be further enhanced. An image portion corresponding to the non-attention region is an image having uniform color (density) and no contrast. The image portion corresponding to the attention region becomes an image having a large concentration change and a clearer contrast.

The image processing system S performs the signal processing on the analog signal before the analog-digital conversion. When the signal processing is performed on a digital signal, fine differences in output values may disappear. By performing the signal processing on the attention region before (prior to) the analog-digital conversion, it is possible to reflect a fine difference of the analog signal in the density in the X-ray image.

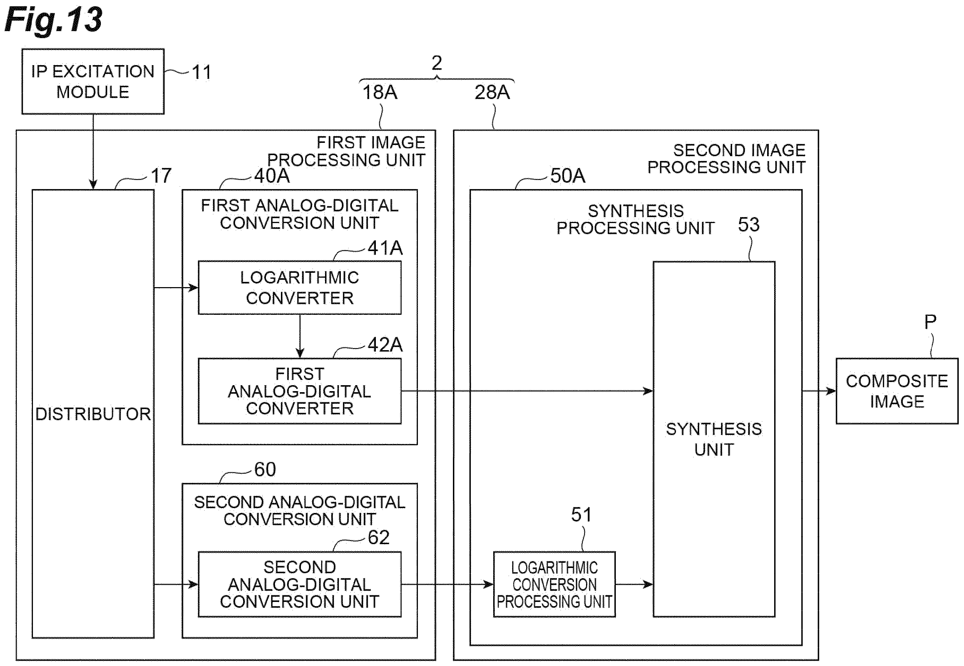

The configuration of the image processing unit may be different from that of the above embodiment. FIG. 13 is a block diagram showing another configuration example in the image processing unit. As shown in FIG. 13, a first image processing unit 18A having a distributor 17, a first analog-digital conversion unit 40A including a logarithmic converter 41A and a first analog-digital converter 42 A, and a second analog-digital conversion unit 60 including a second analog-digital converter 62 may be applied. In that case, a second image processing unit 28A having the synthesis processing unit 50 including a logarithmic conversion processing unit 51 and a synthesis unit 53 may be applied. The logarithmic conversion processing unit 51 may be configured to process the signal from the second analog-digital converter 62 by digital computation. The first 18 The image processing unit and the second 8 The image processing unit 2 constitute an image processing unit 2A. The first analog-digital conversion unit 40A may be constituted by a circuitry and may be expressed as a first analog-digital conversion circuitry 40A. The second analog-digital conversion unit 60 may be configured as a circuitry and may be referred to as a second analog-digital conversion circuitry 60. The logarithmic conversion processing unit 51 may be composed of circuitry and may be described as a logarithmic conversion processing circuitry 51. The synthesis unit 53 may be composed of circuitry and may be described as a synthesis circuitry 53.

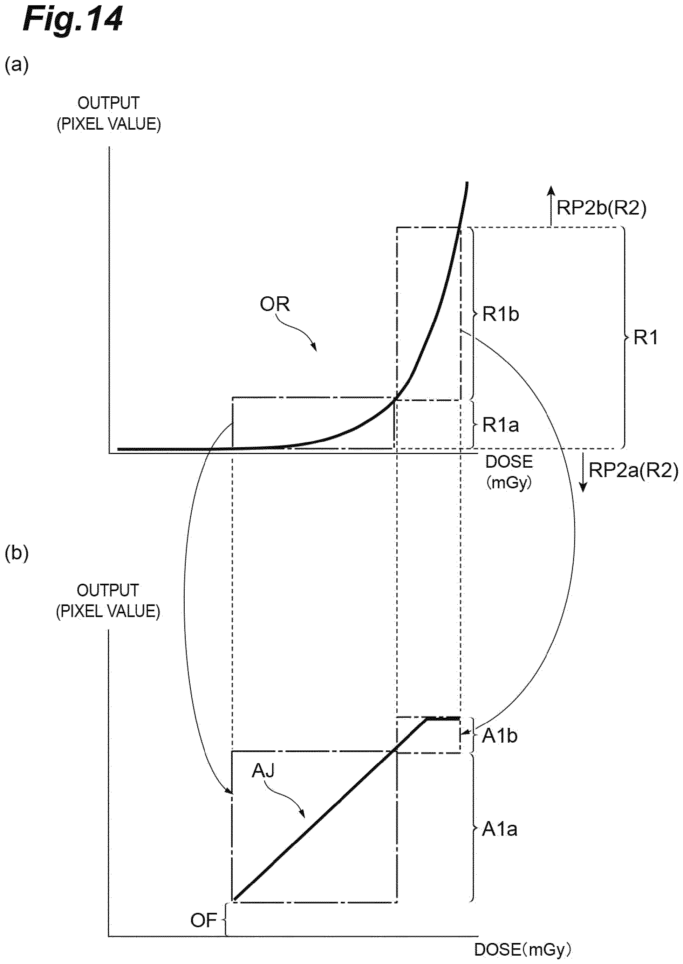

In the image processing system S having the above-described configuration, the first analog-digital conversion unit 40A performs first analog / digital conversion adapted to a signal band having a relatively low dose intensity on the total output area included in the read signal, and the second analog-digital conversion unit 60 performs second analog/digital conversion adapted to a signal band having a relatively high dose intensity. The first analog-digital conversion unit 40A includes the logarithmic converter 41A and performs logarithmic conversion on the read signal. For a signal band having a relatively low dose intensity, since the degree of change in output is small with respect to the degree of change in dose, conversion is performed so that this degree becomes large. This conversion takes place before the analog-digital conversion. The logarithmic converter 41A is an example of this configuration, and the degree of change in the output may be increased by using an element such as an amplifier that converts the gain of the input signal without using the logarithmic converter 41A. Then, the synthesis processing unit 50 performs image processing for synthesizing both converted signals. In this case, image processing is performed by combining the revised value shown in FIG. 10(a) and the revised value shown in FIG. 11(a). Clear contrast can be obtained in both the low dose region RA and the high dose region RB. As shown in FIGS. 14(a) and 14 b, the analog-digital conversion and logarithmic conversion corresponding to each of the first attention region R1a and the second attention region R1b are performed, and an advantageous X-ray image corresponding to each signal band can be obtained. In the adjustment output pattern AJ shown in FIG. 14(b), the concentration is constant in the high dose region. As described above, depending on the diagnostic purpose, a portion where the output change is steep and a portion where the output change is gentle may be mixed in the attention region.

The signal processing performed by the image processing unit 2 will be described with reference to FIGS. 13, 16(a) to 16(c), 17(a), 17(b), 18(a), and 18(b).

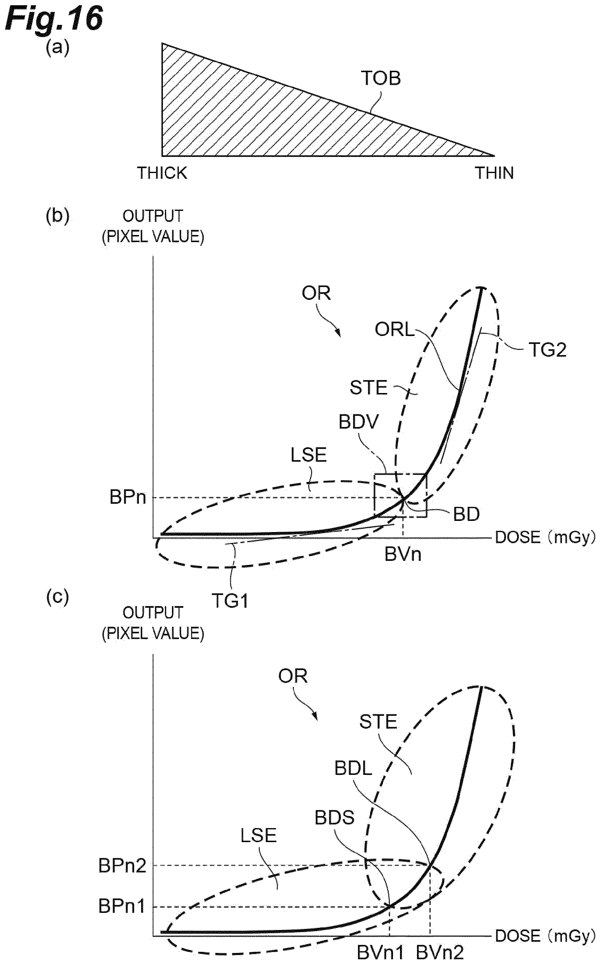

Here, it is assumed that there is an object TOB as shown in FIG. 16 a in which the thicknesses increase like an arithmetic progression from thin to thick. As the object TOB, for example, a material such as aluminum which transmits X-rays to some extent is used. When this the object TOB is irradiated with X-rays, the X-rays are absorbed according to their thicknesses. The amount of received transmitted X-rays on the side of an X-ray detection element such as an IP is small for irradiation of a thick portion and large for irradiation of a thin portion. If this intensity is detected without performing output adjustment, the result is as shown in FIG. 16(b). As the intensity detection, for example, detection of an output of intensity of a signal received from the light receiving unit 16 is considered. In the read signal illustrated in the graph of FIG. 16(b), the left side corresponds to a portion having a large thickness, that is, a low dose, and the right side corresponds to a portion having a small thickness, that is, a high dose. The pattern of the original output with respect to the dose, which is not subjected to the output adjustment processing according to the present configuration described below, is referred to as the original output pattern OR. This the original output pattern OR is indicated by the original output pattern line ORL on the graph as shown in FIG. 16(b).

As can be understood by observing the original output pattern line ORL, in the original output pattern OR, many regions in which the degree of change in the output value with respect to the change in the dose value is small are included in the low dose region, and many regions in which the degree of change in the output value with respect to the change in the dose value is large are included in the high dose region. A region boundary BD (BVn, BPn) is set so that the original output pattern OR is divided into a low change region LSE which is a region on the side where the degree of change in the output value is small and a high change region STE which is a region on the side where the degree of change in the output value is large. This boundary area may be set as a default or may be variable by operation. The region boundary BD may be variably adjustable within a variable range BDV. The distinction between the low change region LSE and the high change region STE may be made according to the magnitude of the inclination of a tangent TG1 when the tangent TG1 is determined for the original output pattern line ORL of the low change region LSE and the inclination of a tangent TG2 when the tangent TG1 is determined for the original output pattern line ORL of the high change region STE, for example, according to whether the inclination is smaller or larger than a reference ratio.

As shown in FIG. 16(c), the low change region LSE and the high change region STE may partially overlap each other. That is, the high output end of the low change region LSE may be higher than the low output end of the high change region STE.

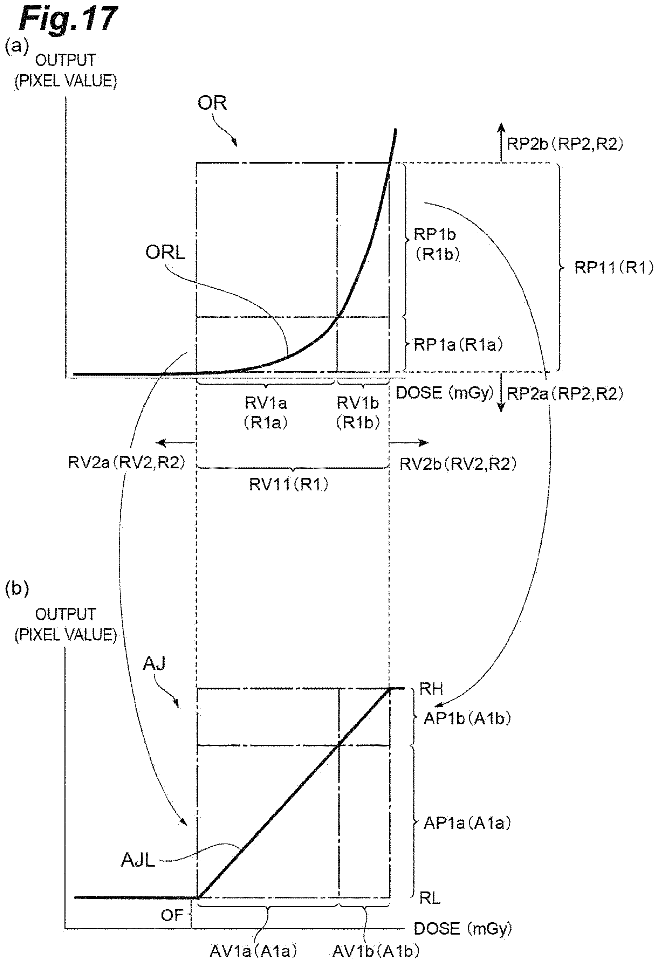

An application example based on the same idea as that of the embodiment shown in FIG. 14 will be described with reference to FIGS. 17(a) and 17(b). FIGS. 17(a) and 17(b) are different from FIGS. 14 a and 14 b only in that the pattern of the second adjustment range R1b with respect to the second output range A1b in FIG. 17(b) is slightly different from that in FIG. 14(b). In FIGS. 17(a) and 17(b) and FIGS. 14(a) and 14(b), the following ideas are common.

In the low dose region, adjustment is performed such that the change becomes steep in a region on the side where the degree of the change in the output value with respect to the change in the dose value is small.

In the high dose region, adjustment is performed such that the change becomes gentle in a region on the side where the degree of change in the output value with respect to the change in the dose value is large.

In order to optimize the degree of change in the original output pattern OR shown in FIG. 16(b), a first output range (first attention region) R1a is set for the low change region LSE and a second output range (second attention region) R1b is set for the high change region STE, as shown in FIG. 17(a), and adjustment processing is performed for each as shown in FIG. 17(b). The adjustment output pattern AJ related to the adjustment processing is indicated by the adjusted output pattern line AJL on the graph as illustrated in FIG. 17(b). As the adjustment processing, the first output range R1a (RV1a, RP1a) is adjusted like the first adjustment range A1a (AV1a, AP1a) so that the degree of change of the output value in the adjustment output pattern AJ is larger than the degree of change of the output value in the original output pattern OR, and the second output range R1b (RV1b, RP1b) is adjusted like the second adjustment range A1b (AV1b, AP1b) so that the degree of change of the output value in the adjustment output pattern AJ is smaller than the degree of change of the output value in the original output pattern OR. When the low change region LSE and the high change region STE partially overlap each other as shown in FIG. 16(c), adjustment is performed so as to have continuity and consistency between the first adjustment area A1a and the second adjustment area A1b.

In FIG. 17, linear processing is performed on the entire second output range R1b like the second adjustment range A1b, but as shown in FIG. 14(b), a uniform output value may be assigned to a part of the high dose side. The same applies to the first output range R1a, and a uniform output value may be assigned to a part of the low dose side. A uniform output value may be assigned to both a part of the low dose side of the first output range R1a and a part of the high dose side of the second output range R1b.

The image processing unit 2 sets the partial attention region R1 (RV11, RP11) according to the purpose of observation and the non-attention region R2 (RV2a+RV2b→RV2, RP2a+RP2b→RP2) other than the attention region R1 in the total output area included in the read signal (see FIG. 17(a)). For example, it can be determined that the first non-attention region RV2a and RP2a having lower output values of the non-attention region R2 are represented by a uniform adjusted output value (for example, white), and the second non-attention region RV2b and RP2b having higher output values are represented by a uniform adjusted output value (for example, black). The offset value OF may be assigned to the first non-attention region RP2a. The attention region R1 may comprise a first output range R1a and a second output range R1b.

FIG. 17(b) shows an example of a linear adjustment pattern. The adjustment output pattern AJ 's the upper limit value RH and the lower limit value RL may be determined as appropriate, but may be adapted to a range between the minimum and maximum values of the attention region R1 in the original output pattern OR as shown.

According to such signal processing (image processing), it is possible to avoid a burden of unnecessary calculation and to effectively use a limited number of bits in calculation of the processor. In the graph, an element on the vertical axis is expressed by insertion of a character P, and an element on the horizontal axis is expressed by insertion of a character V.

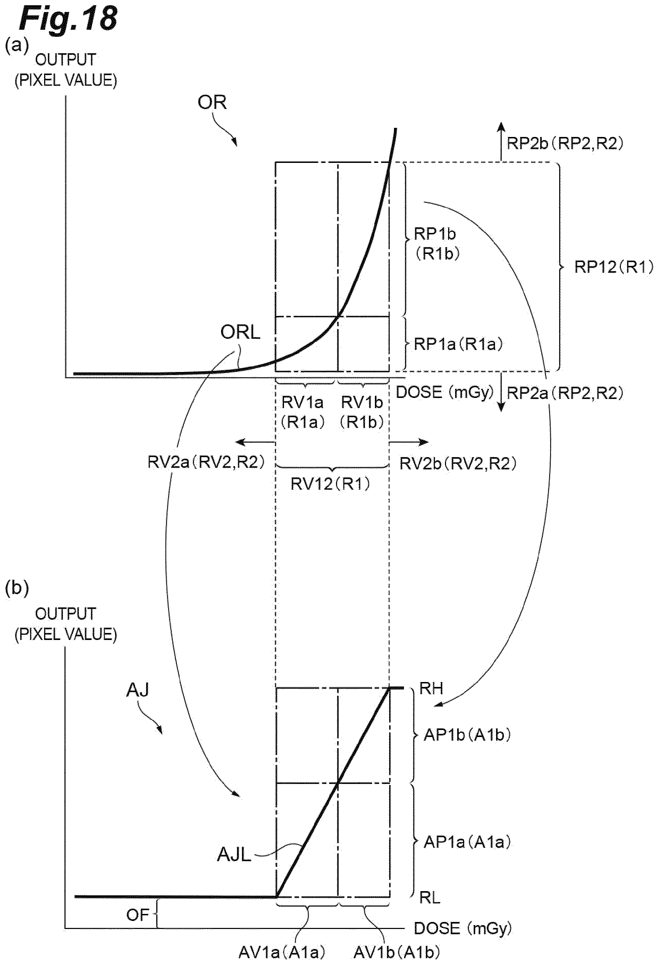

In contrast to a linear adjustment pattern over a wide the attention region R1 as shown in FIG. 17, it is conceivable to limit the attention region R1 and perform the signal processing such that the degree of change in density for the attention region is larger (steeper) than the degree of change in density for the non-attention region. FIG. 18(b) shows an example in which such adjustment processing is performed. In FIG. 18, the range of the attention region RV12 and RP12 (R1) is limited to a higher dose region than the example of R1 shown in FIG. 17. The analog-digital conversion unit 40 performs the signal processing such that the degree of change in density regarding the attention region is larger (steeper) than the degree of change in density regarding the non-attention region. The degree of change in density for the non-attention the attention region is less severe than the degree of change in density for the region. The gradual change includes a case where the degree of change is not 0 and a case where the degree of change is 0. Although the upper limit value RH and the lower limit value RL of the adjustment output pattern AJ can be determined as appropriate, they may be matched with the upper limit value and lower limit value of the adjustment output pattern AJ in FIG. 18 as shown in the figure. The adjustment processing will be described in detail later.

The synthesis unit 53 combines the digital signal converted by the first analog-digital conversion unit 40A and the second analog-digital conversion unit 60 to generate the composite image P. The synthesis unit 53 inputs the digital signal from the first analog-digital converter 42A and the digital signal from the logarithmic conversion processing unit 51, and performs HDR (High Dynamic Range) synthesis.

As described above, in the image processing system S, the first analog-digital conversion unit 40A performs logarithmic conversion and first analog/digital conversion (first signal processing) adapted to a signal band having a relatively low dose intensity on the total output area included in the read signal. Further, the second analog-digital conversion unit 60 performs second analog/digital conversion and logarithmic conversion (second signal processing) adapted to a signal band having a relatively high dose intensity. As described above, the signal processing in the first analog-digital conversion unit 40A is different from the signal processing in the second analog-digital conversion unit 60. Then, a synthesis processing unit 50A performs image processing for synthesizing both converted signals. In this case, image processing is performed by combining the revised values shown in FIG. 10(a) and the revised values shown in FIG. 11(a), and clear contrast can be obtained in both the low dose region RA and the high dose region RB described above. As shown in FIGS. 17(a) and 17(b), the analog-digital conversion and logarithmic conversion corresponding to each of the first output range R1a and the second output range R1b are performed, and an advantageous X-ray image corresponding to each signal band can be obtained.

According to the image processing system S of this embodiment, the first analog-digital conversion unit 40A performs the first signal processing adapted to the first output range R1a (see FIG. 17(a)) having a relatively small output, and the second analog-digital conversion unit 60 performs the second signal processing adapted to the second output range R1b (see FIG. 17(a)) having a relatively large output. The first signal processing converts a signal corresponding to a light color portion in the X-ray image, and the second signal processing converts a signal corresponding to a dark color portion in the X-ray image. By performing the signal processing adapted to each output range and synthesizing them, it is possible to obtain an X-ray image with good contrast regardless of a light color portion and a dark color portion in the X-ray image.

The analog logarithmic conversion has several advantages. That is, in the analog logarithmic conversion, the data can be finely taken in the low-dose region. In addition, after logarithmic conversion appears as it is, no digital noise is generated. In the image processing system S, the first analog-digital conversion unit 40A performs advantageous signal processing in the low-dose region. On the other hand, digital logarithmic conversion also has several advantages. That is, in the digital logarithmic conversion, the data can be finely taken in the high dose region. In addition, electrical noise is not superimposed. In the image processing system S, the second analog-digital conversion unit 60 performs advantageous signal processing in the high dose region.

Also in the image processing system S, the first analog-digital conversion unit 40A performs logarithmic conversion on the analog signal. When the first signal processing is performed on the digital signal, a fine difference in an output value may disappear. Particularly, in a light color portion, a fine output value difference is valuable information. By performing the signal processing on the first output range R1a before (prior to) the analog-digital conversion, it is possible to reflect the difference of the analog signal in density in the X-ray image.

The first analog-digital conversion unit 40A performs the signal processing so that the degree of change in density regarding the first output range R1a becomes larger than the degree of change in density regarding the region other than the first output range R1a, and the second analog-digital conversion unit 60 performs the signal processing so that the degree of change in density regarding the second output range R1b becomes larger than the degree of change in density regarding the region other than the second output range R1b. Therefore, the degree of change in density becomes large for each output range in which the first and second signal processing is performed. As a result, the contrast is improved in the entire obtained X-ray image.

Although the embodiment of the present invention has been described above, the present invention is not limited to the above embodiment. For example, in the first signal processing, logarithmic conversion may be performed on the digital signal after the analog-digital conversion. The second signal processing may include signal processing other than the analog-digital conversion. In the above-described embodiment, the distributor 17 supplies the same read signal for the total output area to each of the first analog-digital conversion unit 40A and the second analog-digital conversion unit 60. However, unlike this, the distributor 17 may supply signals of only the first output range to the first analog-digital conversion unit 40A, and may supply signals of only the second output range to the second analog-digital conversion unit 60. That is, the distributor 17 may supply a read signal to each analog-digital conversion unit so as to distribute only a signal in a signal processable (adaptable) output range that can be advantageously processed by each analog-digital conversion unit.

An image processing system of the present disclosure is not limited to processing a read signal read from the imaging plate on which the radiographic image is recorded. The image processing system of the present disclosure may be the image processing system that processes radiographic detection signals to produce an X-ray image. The detection signal by radiography is, for example, a detection signal output by a CCD, a CMOS, or the like as an imaging element (semiconductor sensor). In this case, the image processing system may perform the signal processing on the total output area included in the detection signal so that the degree of change in density regarding a partial attention region according to the purpose of observation is larger than the degree of change in density regarding the non-attention region other than the attention region. The signal processing disclosed in the above embodiments may be applied to the detection signal output by the imaging element. Even in this case, the degree of change in density is large only for a partial attention region corresponding to the purpose of observation in the total output area included in the detection signal. Therefore, in the obtained X-ray image, the contrast of the portion to be observed is improved.

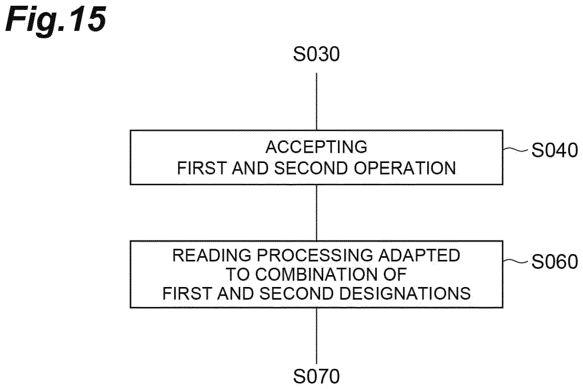

As shown in FIG. 15, in step S040, the main control unit 20 may accept first and second operations (designations). Further, in step S060, the main control unit 20 may read out a processing adapted to a combination of the first and second operations (designations). In this manner, the image processing unit 2 may be configured to enable image processing for multiple purposes.

The technical subject according to the present disclosure can be expressed as the following technical subject 1 to 9 when the mechanical configuration is emphasized.

### [Technical Subject 1]

An image processing circuitry for generating an X-ray image by processing a read signal read from the imaging plate on which a radiographic image is recorded,
wherein signal processing is performed on a total output area included in the read signal such that a degree of change in density regarding a partial attention region according to an observation purpose is greater than a degree of change in density regarding a non-attention region other than the attention region.

### [Technical Subject 2]

The image processing circuitry according to technical subject 1, wherein the signal processing is performed by logarithmic conversion.

### [Technical Subject 3]

The image processing circuitry according to technical subject 1 or 2, wherein the attention region and the non-attention region are set such that a dose intensity range corresponding to the attention region is existing on a lower dose side than a dose intensity range corresponding to the non-attention region.

### [Technical Subject 4]

The image processing circuitry according to technical subject 1 or 2, wherein the attention region and the non-attention region are set such that a dose intensity range corresponding to the attention region is existing on a higher dose side than a dose intensity range corresponding to the non-attention region.

### [Technical Subject 5]

The image processing circuitry according to technical subject 1 or 2, wherein the attention region and the non-attention region are set so that the non-attention region includes a first non-attention region having a dose intensity range existing on a lower dose side than a dose intensity range corresponding to the attention region and a second non-attention region having a dose intensity range existing on a higher dose side than the dose intensity range corresponding to the attention region.

### [Technical Subject 6]

The image processing circuitry according to any one of technical subjects 1 to 5, wherein the signal processing is performed so that the non-attention region is represented with a constant density by eliminating a degree of change in density regarding the non-attention region.

### [Technical Subject 7]

The image processing circuitry according to any one of technical subjects 1 to 6, wherein the signal processing is performed on the analog signal before the analog-digital conversion.

### [Technical Subject 8]

An image processing circuitry that generates an X-ray image by processing a detection signal obtained by radiography,
wherein signal processing is performed on a total output area included in the detection signal such that a degree of change in density regarding a partial attention region according to an observation purpose is greater than a degree of change in density regarding a non-attention region other than the attention region.

### [Technical Subject 9]

An image processing apparatus, wherein each component of the image processing circuitry of any one of technical subjects 1 to 8 is integrated to form a single apparatus.

In the conventional technique described in Patent Document 1, processing such as logarithmic conversion amplification and A/D conversion is performed on a read signal obtained by detection of stimulated emission. In general, in an obtained X-ray image, a portion where the dose intensity of X-rays is small has a light color, and a portion where the dose intensity of X-rays is large has a dark color. An X-ray image is composed of pixels having any density from a light portion to a dark portion. However, in the conventional technique, a blurred image portion may occur at any dose intensity. The blurred image portion is an image portion in which the contrast is not clear and is fuzzy.

The technical subject matter described below relates to the image processing system capable of obtaining an X-ray image with good contrast regardless of whether the color of the X-ray image is light or dark.

The technical subject related to the system and the signal processing described with reference to FIG. 16(a) and the subsequent drawings can be expressed as the following technical subject 1A to 6A when the mechanical configuration is emphasized.

### [Technical Subject 1A]

An image processing system for processing a read signal read from the imaging plate on which a radiographic image is recorded by logarithmic conversion to generate an X-ray image,
wherein an output range included in the read signal includes a predetermined first output range and a second output range having an output larger than that of the first output range,
the image processing system comprising:
   a first analog-digital conversion unit configured to perform first signal processing adapted to the first output range;
   a second analog-digital conversion unit configured to perform second signal processing different from the first signal processing adapted to the second output range; and
   a synthesis unit configured to synthesize digital signals converted by the first image processing system and the second analog-digital conversion unit.

### [Technical Subject 2A]

The image processing system described in the technical subject 1A, wherein the first analog-digital conversion performs the logarithmic conversion on the analog signal.

### [Technical Subject 3A]

Wherein the first analog-digital conversion unit performs signal processing such that a degree of change in density regarding the first output range is greater than a degree of change in density regarding a region other than the output range, and
The image processing system according to technical subject 1A or 2A, wherein the second analog-digital conversion unit performs signal processing such that a degree of change in density for the second output range is greater than a degree of change in density for a region other than the output range.

### [Technical Subject 4A]

An image processing system for processing a radiographic detection signal by logarithmic conversion to generate an X-ray image,
wherein an output range included in the detection signal includes a predetermined first output range and a second output range having an output larger than that of the first output range,
the image processing system comprising:
   a first analog-digital conversion unit configured to perform first signal processing adapted to the first output range;
   a second analog-digital conversion unit configured to perform second signal processing different from the first signal processing adapted to the second output range; and
   a synthesis unit configured to synthesize digital signals converted by the first image processing system and the second analog-digital conversion unit.

### [Technical Subject 5A]

The image processing system according to any one of technical subjects 1A to 4A, wherein in the read signal, a region on a side where a degree of change of an output value with respect to change of a dose value is small in a low dose region is set as a low change region, a region on a side where a degree of change of an output value with respect to change of a dose value is large in a high dose region is set as a high change region, the first output range is set for the low change region, and the second output range is set for the high change region.

### [Technical Subject 6A]

An image processing apparatus, wherein each component of each the image processing system of any one of technical subject 1A to 5A is integrated to form a single device.

According to the image processing system of the technical subject 1A, the first analog-digital conversion unit performs first signal processing adapted to a first output range having a relatively small output, and the second analog-digital conversion unit performs second signal processing adapted to a second output range having a relatively large output. The first signal processing converts a signal corresponding to a light color portion in the X-ray image, and the second signal processing converts a signal corresponding to a dark color portion in the X-ray image. By performing the signal processing adapted to each output range and synthesizing them, it is possible to obtain an X-ray image with good contrast regardless of a light color portion and a dark color portion in the X-ray image.

In the image processing system according to the technical subject 2A, the first analog signal may perform logarithmic conversion on the analog signal. When the first signal processing is performed on the digital signal, a fine difference in an output value may disappear. Particularly, in a light color portion, a fine output value difference is valuable information. By performing the signal processing on the first output range before (prior to) the analog-digital conversion, it is possible to reflect the difference of the analog signal in density in the X-ray image.

In the image processing system according to the technical subject 3A, the first analog-digital conversion unit may perform the signal processing such that the degree of change in density regarding the first output range is greater than the degree of change in density regarding a region other than the first output range, and the second analog-digital conversion unit may perform the signal processing such that the degree of change in density regarding the second output range is greater than the degree of change in density regarding a region other than the second output range. In this case, with respect to each output range in which the first and second signal processing are performed, the degree of change in density becomes large. Therefore, the contrast is improved in the entire obtained X-ray image.

According to the image processing system of the technical subject 4A, the first analog-digital conversion unit performs first signal processing adapted to a first output range having a relatively small output, and the second analog-digital conversion unit performs second signal processing adapted to a second output range having a relatively large output. The first signal processing converts a signal corresponding to a light color portion in the X-ray image, and the second signal processing converts a signal corresponding to a dark color portion in the X-ray image. By performing the signal processing adapted to each output range and synthesizing them, it is possible to obtain an X-ray image with good contrast regardless of a light color portion and a dark color portion in the X-ray image.

According to the image processing system related to the technical subject 5A, in a read signal, a region on a side where a degree of change of an output value with respect to change of a dose value is small in a low dose region is set as a low change region, a region on a side where a degree of change of an output value with respect to change of a dose value is large in a high dose region is set as a high change region, a first output range is set for the low change region, and a second output range is set for the high change region. As a result, the degree of change in the output value can be equalized, and appropriate distribution can be performed.

The technical subject 1A to 5A related to the system and the signal processing described with reference to FIG. 16(a) and subsequent figures can be expressed as the following technical subject 1B to 6B when the mechanical configuration is emphasized.

### [Technical Subject 1B]

An image processing circuitry for generating an X-ray image by performing logarithmic conversion on a read signal read from the imaging plate on which a radiographic image is recorded,
wherein an output range included in the read signal includes a predetermined first output range and a second output range having an output larger than that of the first output range,
the image processing circuitry comprising:
   a first analog-digital conversion circuitry configured to perform first signal processing adapted to the first output range;
   a second analog-digital conversion circuitry configured to perform second signal processing different from the first signal processing adapted to the second output range; and
   a synthesis circuitry configured to synthesize the digital signals converted by the first analog-digital conversion circuitry and the second analog-digital conversion circuitry.

### [Technical Subject 2B]

The image processing circuitry according to technical subject 1B, wherein the first analog-digital conversion circuitry performs the logarithmic conversion on the analog signal.

### [Technical Subject 3B]

The image processing circuitry according to technical subject 1B or 2B, wherein the first analog-digital conversion circuitry performs signal processing such that a degree of change in density regarding the first output range is greater than a degree of change in density regarding a region other than the output range, and
wherein the second analog-digital conversion circuitry performs signal processing such that a degree of change in density for the second output range is greater than a degree of change in density for a region other than the output range.

### [Technical Subject 4B]

An image processing circuitry that generates an X-ray image by processing a detection signal obtained by radiography through logarithmic conversion,
wherein an output range included in the detection signal includes a predetermined first output range and a second output range having an output larger than that of the first output range,
the image processing circuitry comprising:
   a first analog-digital conversion circuitry configured to perform first signal processing adapted to the first output range;
   a second analog-digital conversion circuitry configured to perform second signal processing different from the first signal processing adapted to the second output range; and
   a synthesis circuitry configured to synthesize the digital signals converted by the first analog-digital conversion circuitry and the second analog-digital conversion circuitry. 2.

### [Technical Subject 5B]

The image processing circuitry according to any one of technical subject matters 1B to 4B, wherein in the read signal, a region on a side where a degree of a change in an output value with respect to a change in a dose value is small in a low dose region is a low change region, a region on a side where a degree of a change in an output value with respect to a change in a dose value is large in a high dose region is a high change region, the first output range is set for the low change region, and the second output range is set for the high change region.

### [Technical Subject 6B]

An image processing apparatus, wherein each component of the image processing circuitry of any one of technical subject 1B to 5B is integrated to form a single device.

### Reference Signs List

2...image processing unit, 3...display unit, 4...operation unit, 10...first image processing apparatus, 18...first image processing unit, 20...main control unit, 30...second image processing apparatus, 28...second image processing unit, 50...synthesis processing unit, R1...the attention region, R2...non-attention region, S...the image processing system.

## Claims

1. An image processing system for processing a read signal read from an imaging plate on which a radiographic image is recorded to generate an X-ray image,
wherein signal processing is performed on a total output area included in the read signal such that a degree of change in density regarding a partial attention region according to an observation purpose is greater than a degree of change in density regarding a non-attention region other than the attention region.

2. The image processing system according to claim 1, wherein the signal processing is performed by logarithmic conversion.

3. The image processing system according to claim 1 or 2, wherein the attention region and the non-attention region are set such that a dose intensity range corresponding to the attention region is existing on a lower dose side than a dose intensity range corresponding to the non-attention region.

4. The image processing system according to claim 1 or 2, wherein the attention region and the non-attention region are set such that a dose intensity range corresponding to the attention region is existing on a higher dose side than a dose intensity range corresponding to the non-attention region.

5. The image processing system according to claim 1 or 2, wherein the attention region and the non-attention region are set such that the non-attention region includes a first non-attention region having a dose intensity range existing on a lower dose side than a dose intensity range corresponding to the attention region and a second non-attention region having a dose intensity range existing on a higher dose side than the dose intensity range corresponding to the attention region.

6. The image processing system according to any one of claims 1 to 5, wherein the signal processing is performed such that the non-attention region is represented with a constant density by eliminating a degree of change in density regarding the non-attention region.

7. The image processing system according to any one of claims 1 to 6, wherein the signal processing is performed on an analog signal before an analog-digital conversion.

8. An image processing system for processing a radiographic detection signal to generate an X-ray image,
wherein signal processing is performed on a total output area included in the detection signal such that a degree of change in density regarding a partial attention region according to an observation purpose is greater than a degree of change in density regarding a non-attention region other than the attention region.

9. An image processing apparatus wherein each component of the image processing system of any one of claims 1 to 8 is integrated to form a single apparatus.
